(19) Europäisches Patentamt
European Patent Office
Office européen des brevets

(11) Numéro de publication : **0 006 782**
**B1**

(12) **FASCICULE DE BREVET EUROPÉEN**

(45) Date de publication du fascicule du brevet : **17.06.81**

(51) Int. Cl.³ : **C 07 D487/04** // (C07D487/04, 235/00)

(21) Numéro de dépôt : **79400351.7**

(22) Date de dépôt : **01.06.79**

(54) **Procédé de fabrication de dinitroglycoiurile en continu.**

(30) Priorité : **23.06.78 FR 7818785**

(43) Date de publication de la demande : **09.01.80 (Bulletin 80/01)**

(45) Mention de la délivrance du brevet : **17.06.81 Bulletin 81/24**

(84) Etats contractants désignés : **BE CH DE GB IT LU NL SE**

(56) Documents cités :
**RECUEIL DES TRAVAUX CHIMIQUES DES PAYS-BAS, tome VIII, 1889, Leide (NL) A.P.N. FRANCHIMONT et al. « Contributions à la connaissance de l'action de l'acide azotique sur les corps organiques », pages 283-306.**

(73) Titulaire : **SOCIETE NATIONALE DES POUDRES ET EXPLOSIFS
12, quai Henri IV
F-75181 PARIS CEDEX 04 (FR)**

(72) Inventeur : **Emeury, Jean-Marie
rue des Rosiers
F-84700 Sorgues (FR)**
Inventeur : **Girardon, Hubert
rue Pierre Curie
F-30400 Villeneuve Les Avignon (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 17, rue du Louvre, 75001 Paris, France

EP 0 006 782 B1

Procédé de fabrication de dinitroglycolurile en continu

La présente invention a pour objet un procédé de préparation en continu du dinitroglycolurile.

Le dinitroglycolurile a été synthétisé pour la première fois par FRANCHIMONT et KLOBBIE (Recueil des Travaux Chimiques des Pays-Bas, volume 7, page 188) en 1888. Ces auteurs attribuèrent tout d'abord une formule évidemment erronée au produit qu'ils avaient obtenu par dissolution d'une partie en poids de glycolurile dans cinq parties en poids d'acide nitrique absolu, puis émirent l'hypothèse (Op. cit., pages 246-247) que le dinitroglycolurile résultant de cette opération devait avoir pour formule :

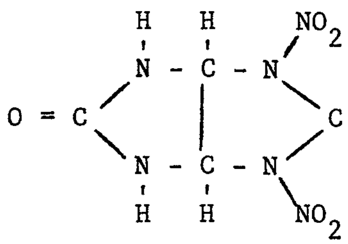
(isomère 1,3)

du fait de la nature des produits de la décomposition du composé réalisée dans l'eau bouillante.

Toutefois, il est bien clair que deux autres isomères peuvent se former, de formules :

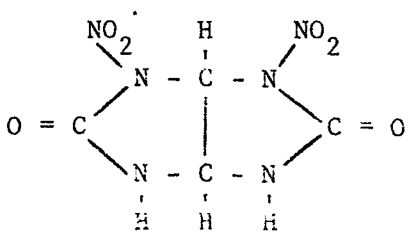
(isomère 1,6)

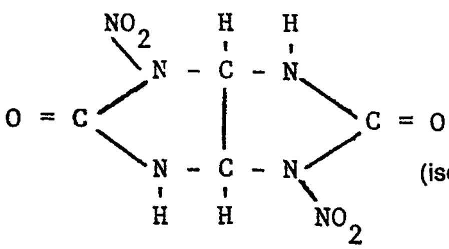
(isomère 1,4)

Ces mêmes auteurs (op. cit. volume 8, pages 290 et 291) ont d'ailleurs mis en évidence l'existence d'au moins un de ces isomères, sans toutefois indiquer comment en favoriser la formation.

Longtemps après que le dinitroglycolurile et ses isomères soient tombés dans l'oubli, la Demanderesse a démontré dans son brevet français 2 238 703 l'intérêt de ce composé en tant qu'explosif secondaire, puis, dans son brevet français 2 379 498, en tant qu'additif énergétique améliorant considérablement les propriétés des hexolites ou compositions « B ».

Il existe donc un besoin important en un procédé de production en masse de dinitroglycolurile (ou DINGU) utilisable comme explosif.

Avantageusement un tel procédé devrait être continu.

Si l'on applique le seul procédé décrit avec relativement de précisions par FRANCHIMONT et KLOBBIE (Op. cit., volume 7) une proportion importante d'isomère 1,3 du DINGU, décomposable par l'eau bouillante, est formée. Or cet isomère est nettement moins stable que les deux autres isomères (1,4 et 1,6) si bien que pour certaines applications il est nécessaire de faire subir un traitement à l'eau bouillante au produit brut de réaction de manière à ne récupérer que les isomères stables du DINGU. Il en résulte une baisse très sensible du rendement global de la nitration.

En revanche, la présence de cette phase homogène a paru extrêmement favoriser la cinétique de la réaction de nitration du glycolurile, tous isomères confondus.

Confrontée à ces observations inédites et surprenantes, la Demanderesse a maintenant trouvé un procédé de fabrication de DINGU fonctionnant en continu et favorisant la formation des isomères du DINGU les plus stables.

Le procédé selon l'invention consiste à effectuer la synthèse du dinitroglycolurile par nitration de glycolurile à l'aide d'acide nitrique absolu et est caractérisé en ce qu'on effectue la nitration en continu et en cascade, en réalisant une phase liquide homogène par introduction simultanée et continue de glycolurile et d'acide nitrique absolu dans un premier réacteur agité, puis une phase hétérogène dans un second réacteur agité d'une manière suffisante pour maintenir en mouvement la suspension cristalline de DINGU dans le mélange réactionnel liquide.

Le succès du procédé est essentiellement lié au respect des conditions caractéristiques qui viennent d'être énoncées. Toutefois la Demanderesse a également trouvé qu'il existait des conditions de marche plus restrictives conduisant à un fonctionnement particulièrement avantageux. Deux facteurs de grande importance sont le rapport initial de nitration et la température régnant dans les deux étapes de la cascade.

Selon une première variante préférée de réalisation du procédé selon l'invention on utilise un rapport de nitration initial compris entre 4 et 8, de préférence entre 5 et 7, le rapport de nitration initial étant le rapport des masses d'acide nitrique absolu et de glycolurile introduites dans le premier réacteur par unité de temps. L'acide nitrique utilisé dans le cadre du présent procédé doit être absolu c'est-à-dire qu'il doit contenir de 95 à 100 % en poids d'$HNO_3$.

Selon une seconde variante du procédé selon l'invention on fait régner dans le premier réacteur une température comprise entre 25 et 50 °C, de préférence comprise entre 30 et 40 °C. Dans le second réacteur on fait régner une température comprise entre 45 et 70 °C, de préférence comprise entre 50 et 65 °C. Au-dessus de 70 °C, la

réaction de nitration est certes possible mais s'accompagne de phénomènes d'oxydation. Au dessous de 45 °C la réaction de nitration en phase hétérogène est relativement lente, ce qui allonge inutilement le temps de séjour dans le réacteur et la dimension de ce dernier, ce qui n'est pas souhaitable pour des raisons de sécurité et de rentabilité.

Selon une troisième variante du procédé on fait succéder aux deux premiers réacteurs en cascades précités au moins un troisième réacteur agité dans lequel la température du mélange réactionnel est ramenée au voisinage de la température ordinaire et dans lequel la nitration est au besoin achevée. Selon la présente variante le mélange réactionnel est finalement filtré et la masse cristallisée obtenue lavée jusqu'à neutralité, éventuellement claircée et séchée. Le lavage jusqu'à neutralité peut être effectué à l'eau froide jusqu'à obtention de pH5, le clairçage éventuel à l'aide de méthanol ou d'éthanol et le séchage à 65 °C environ. Si la présente variante n'est pas utilisée le mélange réactionnel ne se déverse pas en cascade dans le troisième réacteur mais sur un filtre à chaud où le produit filtré subit le traitement qui vient d'être décrit, de préférence après refroidissement.

Bien que le procédé selon l'invention permette de réduire considérablement l'obtention, dans le mélange réactionnel final, de produits peu stables (vraisemblablement isomère 1,3 du DINGU), on peut, si on le désire, faire subir au produit filtré, lavé et éventuellement claircé ou au produit simplement filtré un lavage à l'eau bouillante.

Enfin, dans ce qui précède, il a été précisé que les deux ou trois réacteurs disposés en cascades selon le procédé selon l'invention sont agités. L'agitation dans le premier réacteur doit être suffisante pour permettre une dissolution rapide du glycolurile dans l'acide nitrique introduit simultanément ou dans le mélange réactionnel déjà contenu dans le réacteur. Dans le second réacteur l'agitation doit être suffisante pour maintenir en mouvement la suspension cristalline de DINGU dans le mélange réactionnel liquide : si un rapport de nitration inférieur à 5 est choisi, une agitation puissante est généralement nécessaire pour atteindre le régime précité. Enfin, dans le dernier réacteur, facultatif, une agitation similaire à celle régnant dans le second est avantageusement utilisée, permettant éventuellement en outre la vidange par trop plein dudit réacteur au fur et à mesure de son remplissage.

La mise en marche ou l'arrêt d'une installation mettant en œuvre le procédé selon l'invention n'offre pas de difficultés particulières. Lors de la mise en marche on pourra par exemple remplir totalement le premier réacteur et à moitié le second réacteur, à l'aide d'acide nitrique absolu. Lors de l'arrêt de l'installation il suffit de couper les alimentations en réactifs frais, de porter les deux premiers réacteurs à la température requise pour le second en marche normale, de laisser mûrir les contenus desdits deux réacteurs et finalement de vidanger ces derniers sur filtre.

En ce qui concerne les temps de séjour dans les deux premiers réacteurs ils sont réglés conformément à l'invention de manière à ce qu'une phase homogène soit présente dans le premier réacteur et une phase hétérogène dans le second réacteur.

Il est à noter que le présent procédé pourrait utiliser de l'oléum nitrique c'est-à-dire de l'acide nitrique à 100 % additionné de $N_2O_5$. Toutefois, il n'y aurait pas d'avantage inattendu à procéder de la sorte mais au contraire risque de formation de SORGUYL (tétranitroglycolurile) lequel serait finalement hydrolysé lors du lavage aqueux ou du traitement à l'eau bouillante finaux.

Le rendement du procédé selon l'invention est compris entre 75 et 95 %, ce qui est au moins comparable au rendement du procédé classique discontinu en phase homogène. Par contre, le produit brut de réaction filtré, lavé à l'eau froide, claircé à l'alcool puis séché, titre généralement plus de 12 % d'azote et contient moins de 5 % en poids de dérivés instables hydrolysables à l'eau chaude, alors qu'en procédant uniquement en phase homogène (en discontinu ou en continu) on obtient au moins 10 % de dérivés instables causes d'insécurité : en effet, si le produit obtenu par le procédé selon l'invention a une stabilité sous vide excellente (moins de 2 $cm^3$ de gaz dégagés par gramme de DINGU au bout de 100 heures à 130 °C), il n'en est pas de même pour le produit obtenu en phase homogène (plus de 15 $cm^3/g$ en 24 heures à 130 °C).

Les exemples suivants sont donnés à titre d'illustration non limitative du procédé selon l'invention ; on y trouvera en outre une comparaison entre le procédé classique en phase homogène, rendu continu de manière inédite et le procédé selon l'invention.

Exemple 1 : Procédé selon l'invention

On a utilisé trois réacteurs de volumes utiles respectifs 170, 500 et 330 ml disposés en cascades. Ces réacteurs étaient équipés d'un tube concentrique permettant à la fois le passage du bras de l'agitateur et l'introduction soit des réactifs (cas du premier réacteur) soit du mélange réactionnel se déversant du réacteur précédent (cas des deuxième et troisième réacteurs). Ainsi le mélange réactionnel était introduit par le centre du réacteur et, après agitation, remontait le long des parois du réacteur jusqu'à un orifice de déversement soit dans le réacteur suivant (cas des premier et deuxième réacteurs), soit sur un filtre (cas du troisième réacteur).

Un distributeur de solide, constitué d'une trémie tronçonique comportant à sa base un orifice donnant sur un axe à alvéoles entraîné par un moteur électrique a permis d'assurer un débit de 200 g/h de glycolurile. L'acide nitrique absolu (à 98 %) a été introduit à raison de 1 200 g/h, soit un rapport de nitration égal à 6.

L'agitation fixée à 500 t/mn dans chaque réacteur a été trouvée convenable pour assurer l'obtention rapide d'un mélange homogène dans

le premier réacteur. Le maintien en mouvement de la suspension de cristaux de DINGU dans le second et le troisième réacteur a été obtenu en augmentant l'agitation progressivement de 500 t/mn à 850-900 t/mn (vitesse de croisière).

La température régnant dans le premier réacteur a fluctué entre 30 et 40 °C. Celles régnant dans les second et troisième réacteur ont respectivement été fixées à 70 et 25 °C.

Les temps de séjour dans les trois réacteurs ont été respectivement de 10, 30 et 20 minutes.

L'installation a fonctionné pendant plusieurs heures au terme desquelles le rendement moyen en DINGU brut a été trouvé égal à 90 %.

Le produit de réaction filtré, puis lavé à l'eau froide jusqu'à obtention d'eaux de lavages à pH5, puis claircé au méthanol et finalement séché à 65 °C, avait une pureté de 95,5 %. Sa stabilité sous vide était de 1,4 $cm^3$ de gaz dégagés par gramme en 100 heures à 130 °C et le taux d'azote de 11,99 %. Ce dingu traité à l'eau bouillante n'a perdu que 4 % de son poids et n'a pas vu sa stabilité sous vide sensiblement améliorée.

Exemple 2 : Procédé classique appliqué en continu

D'une manière inédite on a fait fonctionner en continu le procédé de FRANCHIMONT et KLOBBIE. Dans un réacteur de volume utile de 1,4 litre analogue dans son principe à l'un de ceux de l'exemple 1, c'est-à-dire autorisant l'introduction en continu des réactifs, l'agitation de leur mélange et le déversement en continu du produit de la réaction par un trop plein on a introduit 200 g/h de glycolurile et 2 000 g/h d'acide nitrique à 98,9 %. Le temps de séjour étant de 60 minutes et l'agitation étant fixé à 500 t/mn on a obtenu un mélange réactionnel de sortie homogène. La température dans le réacteur a été maintenue à 55 °C. Le mélange réactionnel se déversant a été reçu dans l'eau glacée d'un cristallisoir. Le précipité, de formation assez lente, a été recueilli sur filtre puis a été lavé à l'eau jusqu'à pH5, claircé au méthanol et séché à 65 °C.

On a obtenu du DINGU brut avec un rendement de 83,6 %. Ce DINGU avait un taux d'azote de 11,8 % et une stabilité sous vide de 15 $cm^3$/g en 24 heures à 130 °C.

Traité à l'eau bouillante, le DINGU obtenu par son procédé perd 20 % de son poids : par contre, le produit résultant a une stabilité sous vide de 2,5 $cm^3$/g en 100 heures à 130 °C.

On voit donc que le procédé selon l'invention permet d'obtenir du DINGU stable avec un rendement meilleur que le procédé dérivé de l'art antérieur, tout en permettant l'économie d'une opération d'hydrolyse à chaud.

Exemple 3: Procédé selon l'invention à l'échelle semi-industrielle

On a utilisé trois nitreurs en cascades ayant respectivement un volume nominal de 60 litres et un volume utile de 50 litres à l'aide d'un doseur à vis « DOSAPRO » on a introduit 15 kg/h de glycolurile. L'acide nitrique (98,8 % $HNO_3$) ayant été introduit à raison de 90 kg/h, le rapport de nitration était sensiblement égal à 6.

Les températures régnant dans les réacteurs étaient respectivement de 30-40 °C (variable), 66 ± 1 °C et 20 ± 2 °C. Ces températures ont été facilement maintenues en faisant circuler de l'eau à 30 °C et à 76 °C respectivement dans la double enveloppe du premier et du second nitreur et de la saumure à − 10°C dans celle du troisième réacteur.

La durée totale de l'opération a été de 10 heures. Au départ on a rempli entièrement le premier réacteur et à moitié le second avec l'acide nitrique à 98,8 %. En fin d'opération, le premier nitreur a été porté à 66 ± 1 °C pendant une heure, de même que le second nitreur : le contenu des trois réacteurs a été finalement vidangé sur filtre.

Le DINGU brut de synthèse a été lavé jusqu'à obtention de pH6 dans les eaux de lavage. On a finalement recueilli au total 313 kg de DINGU à 28,7 % d'humidité, soit 223 kg de DINGU sec. Le rendement de la nitration a donc été de 91 %.

Un échantillon de 500 g de ce DINGU a été soumis à un lavage à l'eau bouillante : on a finalement recueilli 477,5 g de DINGU présentant une excellente stabilité sous vide de 0,89 $cm^3$/g en 130 heures à 100 °C. Le taux d'azote de ce produit était de 12,11 % (théorie : 12,06 %). On en déduit que le DINGU brut de réaction contenait moins de 5 % en poids de produits instables de nitration du glycolurile, hydrolysables dans l'eau chaude.

**Revendications**

1. Procédé de fabrication de dinitroglycolurile par nitration du glycolurile à l'aide d'acide nitrique absolu, caractérisé en ce qu'on effectue la nitration en continu et en cascades, en réalisant une phase liquide homogène par introduction simultanée et continue de glycolurile et d'acide nitrique absolu dans un premier réacteur agité puis une phase hétérogène dans un second réacteur agité d'une manière suffisante pour maintenir en mouvement la suspension cristalline du dinitroglycolurile obtenu dans le mélange réactionnel liquide.

2. Procédé selon la revendication 1, caractérisé en ce qu'on utilise un rapport de nitration initial compris entre 4 et 8, de préférence entre 5 et 7, le rapport de nitration initial étant le rapport des masses d'acide nitrique absolu et de glycolurile introduites dans le premier réacteur par unité de temps.

3. Procédé selon l'une quelconque des revendications précédentes, caractérisé en ce qu'on fait régner dans le premier réacteur une température comprise entre 25 et 50 °C, de préférence entre 30 et 40 °C et, dans le second réacteur, une température comprise entre 45 et 70 °C, de préférence entre 50 et 65 °C.

4. Procédé selon l'une quelconque des revendications précédentes, caractérisé en ce qu'on

fait succéder aux deux premiers réacteurs en cascades au moins un troisième réacteur agité dans lequel la température du mélange réactionnel est ramenée au voisinage de la température ordinaire.

**Claims**

1. Process for the manufacture of dinitroglycoluril by nitration of glycoluril with the aid of absolute nitric acid, characterised in that the nitration is carried out continuously and in series by setting up a homogenous liquid phase in a first stirring vessel by simultaneous and continuous introduction of glycoluril and absolute nitric acid thereinto, and then setting up a heterogenous phase in a second stirring vessel so as to maintain the crystalline suspension of dinitroglycoluril obtained in motion in the liquid reaction mixture.

2. Process according to claim 1, characterised in that an initial nitration ratio between 4 and 8, and preferably 5 and 7, is used, the initial nitration ratio being the ratio of the masses of absolute nitric acid and glycoluril introduced into the first vessel at unit time.

3. Process according to any one of the preceding claims, characterised in that a temperature between 25 and 50 °C, preferably between 30 and 40 °C, prevails in the first vessel, and in that a temperature between 45 and 70 °C, preferably between 50 and 65 °C, prevails in the second vessel.

4. Process according to any one of the preceding claims, characterised in that the process is carried out in two first vessels arranged in series by means of a third stirring vessel in which the temperature of the reaction mixture is reduced to the region of normal temperature.

**Ansprüche**

1. Verfahren zur Herstellung von Dinitroglycoluril durch Nitrierung von Glycoluril mit absoluter Salpetersäure, dadurch gekennzeichnet, daß die Nitrierung kontinuierlich in einer Kaskade durchgeführt wird, wobei durch gleichzeitige und kontinuierliche Einführung von Glycoluril und absoluter Salpetersäure in einen ersten Rührreaktor eine homogene flüssige Phase und anschließend in einem zweiten Reaktor ein heterogenes System erzeugt wird, der hinreichend stark gerührt wird, daß die kristalline Suspension des erhaltenen Dinitroglycolurils im flüssigen Reaktionsgemisch in Bewegung bleibt.

2. Verfahren nach Anspruch 1, dadurch gekennzeichnet, daß ein anfängliches Nitrierverhältnis, das dem Gewichtsverhältnis von pro Zeiteinheit in den ersten Reaktor eingeführter absoluter Salpetersäure zu pro Zeiteinheit in den ersten Reaktor eingeführtem Glycoluril entspricht, von 4 bis 8 und vorzugsweise bis 7 angewandt wird.

3. Verfahren nach Anspruch 1 oder 2, dadurch gekennzeichnet, daß im ersten Reaktor eine Temperatur von 25 bis 50 °C und vorzugsweise 30 bis 40 °C und im zweiten Reaktor eine Temperatur von 45 bis 70°C und vorzugsweise 50 bis 65 °C eingestellt wird.

4. Verfahren nach einem der Ansprüche 1 bis 3, dadurch gekennzeichnet, daß eine Kaskade verwendet wird, der mindestens ein dritter Rührkessel angeschlossen ist, in dem die Temperatur des Reaktionsgemischs in die Nähe der Umgebungstemperatur gebracht wird.